# EUROPÄISCHE PATENTSCHRIFT

(11) **EP 0 876 503 B1**
(45) Veröffentlichungstag und Bekanntmachung des Hinweises auf die Patenterteilung: **13.02.2002**
(21) Anmeldenummer: 96946019.5
(22) Anmeldetag: 27.11.1996
(51) Int. Cl.: C12Q 1/02, G01N 33/50, C12Q 1/68

(54) **VERFAHREN ZUR BEURTEILUNG DER AKTIVITÄT VON ARZNEISTOFFEN**
PROCESS FOR ASSESSING THE ACTIVITY OF DRUGS
PROCEDE D'EVALUATION DE L'ACTIVITE DE SUBSTANCES MEDICAMENTEUSES

(30) Priorität: 28.11.1995 DE 19544333
(43) Veröffentlichungstag der Anmeldung: 11.11.1998
(73) Patentinhaber: Deutsches Krebsforschungszentrum Stiftung des öffentlichen Rechts, 69120 Heidelberg (DE)
(72) Erfinder: DEBATIN, Klaus-Michael, D-69120 Heidelberg (DE); FRIESEN, Claudia, D-69226 Nussloch (DE); KRAMMER, Peter, D-69120 Heidelberg (DE); HERR, Ingrid, D-75015 Bretten (DE)
(74) Vertreter: Schüssler, Andrea, Dr.
(86) Internationale Anmeldenummer: DE9602275
(87) Internationale Veröffentlichungsnummer: WO9720064

(56) Entgegenhaltungen:
- EP-A- 0 675 200
- WO-A-95/10540
- WO-A-95/27735
- WO-A-96/32935
- NATURE, Bd. 373, 2.Februar 1995, Seiten 444-448, XP002030470 JU, S-T. ET AL: "Fas(CD95)/FasL interactions required for programmed cell death after T-cell activation"
- NATURE, Bd. 373, 2.Februar 1995, XP002030471 BRUNNER, T. ET AL: "Cell-autonomous Fas (CD95)/Fas-ligand interaction mediates activation-induced apoptosis in T-cell hybridomas "
- NATURE, XP002030472 LOWIN, B. ET AL: "Cytolytic T-cell cytotoxity through perforin and Fas lytic pathways"

## Beschreibung

Die vorliegende Erfindung betrifft ein Verfahren zur Beurteilung der Aktivität, insbesondere der zytostatischen Aktivität, von Arzneistoffen.

Bisher hat man sich vergeblich darum bemüht, ein System zu entwickeln, mit dem die Aktivität von Arzneistoffen, z.B. gegen Krebs oder AIDS, abgeschätzt werden kann. Man hat beobachtet, daß einige Arzneistoffe, obwohl deren Wirksamkeit z.B. bei bestimmten Krebsarten bekannt war, bei anderen Krebsarten nicht wirksam sind. Im Einzelfall war die Wirksamkeit sogar patientenabhängig und erst unter der Therapie konnte bei Inkaufnahme aller Nebenwirkungen der Arzneistoffe abgeschätzt werden, ob ein Therapieerfolg eintritt oder nicht. Dadurch kam es oft zu Zeitverlusten und die Therapie mußte auf halbem Weg abgebrochen werden und ein neuer Arzneistoff am Patienten ausgetestet werden, von dem man wieder nicht sicher war, ob er den erwünschten Erfolg bringt. Daher besteht ein Bedarf nach einem Verfahren mit dem die Aktivität von Arzneistoffen zuverlässig abgeschätzt werden kann.

Der vorliegenden Erfindung liegt somit die Aufgabe zugrunde, ein Verfahren bereitzustellen, mit dem die Aktivität, insbesondere die zytostatische Aktivität, von Arzneistoffen bestimmt werden kann.

Erfindungsgemäß wird dies durch die Gegenstände in den Patentansprüchen erreicht.

Von der Anmelderin wurde erkannt, daß sich für die Abschätzung der Aktivität von Arzneistoffen, beispielsweise der zytostatischen Aktivität, das Ausmaß von Apoptose eignet, die in Apoptose-positiven Zellen durch die Arzneistoffe induziert wird. Apoptose ist die Bezeichnung für den programmierten Zelltod. Dieser findet sich z.B. bei der Organentwicklung und Metamorphose, der Gewebeatrophie und Tumorregression. Apoptose ist mit einer Kondensation des Cytoplasmas, einem Verlust von Plasmamembranvilli, einer Segmentation des Kerns und extensivem Abbau chromosomaler DNA verbunden. Es hat sich gezeigt, daß eine gesteigerte Apoptose bei verschiedensten Erkrankungen, wie AIDS und Autoimmunerkrankungen, auftritt. Bei AIDS scheint Apoptose für die starke Abnahme der CD4-T Zellen verantwortlich zu sein. Bei Tumoren und Leukämien besteht eine verminderte Apoptose. Forschungsergebnisse der Anmelderin haben gezeigt, daß der zytotoxische Effekt von Arzneistoffen, die in der Tumortherapie eingesetzt werden, sog. Zytostatika, u.a. auf der Induktion von Apoptose beruht.

EP-A-0 675 200 beschreibt ein Verfahren zur Identifizierung von Substanzen, die die Wechselwirkung von CD95 und CD95-Ligand beeinflussen. Das Verfahren erfordert die Inkubation der zu testenden Substanz mit (a) CD95 exprimierenden Zellen in Gegenwart von (b) CD95-L. CD95 -L kann hierbei direkt oder als Kulturüberstand von transformierten, CD95-L exprimierenden Zellen zugesetzt werden. In einer alternativen Ausführungsform werden dem Testansatz transformierte, CD95-L exprimierende Zellen zugegeben.

In vielen normalen und malignen Zellen findet sich ein mit CD95 bzw. APO-1 bezeichnetes Zelloberflächenprotein. An dieses Zelloberflächenprotein kann ein mit CD95-Ligand bzw. APO1-Ligand bezeichnetes lösliches oder Membran-gebundendes Protein binden und die Induktion von Apoptose auslösen.

Durch Verabreichung eines Arzneistoffes, z.B. eines Zytostatikums, wird die Expression von CD95-Ligand, nachstehend mit CD95-L bezeichnet, induziert, der von einer Zelle nach außen freigegeben wird und dann an den in der Zellmembran befindlichen Rezeptor CD95 binden kann. Durch die Bindung von CD95-L an CD95 wird über einen Signalweg Apoptose induziert und es kommt zum Absterben der Zelle (sog. autokriner Selbstmord). Außerdem wird CD95-Ligand nach außen freigegeben und kann an CD95-positive Nachbarzellen binden und damit Apoptose auslösen (sog. Brudermord oder parakriner Tod). Es kommt zu einer Kaskadenwirkung, bei der es zur Auflösung eines ganzen Zellverbandes kommen kann.

Das erfindungsgemäße Verfahren nutzt das CD95/CD95-L System, d.h. es umfaßt die Inkubation von Zellen mit Arzneistoffen sowie den Nachweis des Ausmaßes an Apoptose, die über das CD95/CD95-L-System hervorgerufen wird.

Der Ausdruck "Zellen" umfaßt jegliche Zellen, in denen Apoptose durch das CD95/CD95-L-System hervorgerufen wird. Dies sind vorzugsweise leukämische Zellen, besonders bevorzugt einer T-ALL-Zellinie, ganz besonders bevorzugt die Zellinien CEM oder Jurkat.
Der Ausdruck "Arzneistoffe" umfaßt jegliche bei Mensch oder Tier zur Diagnose und/oder Therapie verwendbare Stoffe. Vorzugsweise sind Arzneistoffe hier zytostatisch wirkende Stoffe, sog. Zytostatika.

Der Nachweis des Ausmaßes an Apoptose kann über verschiedene Wege erfolgen Beispielsweise sind zu nennen:
- morphologische Veränderungen der Zellen, wie Verlust von Plasmamembranvilli oder Segmentation des Kerns oder extensiver Abbau chromosomaler DNA
- DNA-Fragmentationsmuster
- FSC/SSC-Analyse
- hypodiploide Kerne
- Bestimmung der Menge von CD95-L mRNA durch Northern Blot oder RT-PCR.

Der Nachweis der Apoptose über die morphologischen Veränderungen der Zellen kann durch übliche Verfahren, beispielsweise mittels Mikroskopie, erfolgen.

Der Nachweis der Apoptose über das DNA-Fragmentationsmuster kann durch übliche Verfahren erfolgen. Beispielsweise wird aus den mit einem Arzneistoff behandelten Zellen gemäß bekannter Verfahren DNA gewonnen. Diese DNA wird auf einem Agarosegel, z.B. 1,7 % Agarose, elektrophoretisch, z.B. bei 30 Volt für 6 Stunden, aufgetrennt. Je nach Grad der induzierten Apoptose zeigt sich nach Ethidiumbromid-Anfärbung und Visualisierung unter UV-Licht eine mehr oder weniger abgebaute DNA. Bei fortgeschrittener Apoptose zeigt die DNA eine Vielzahl von Banden, die vom Auge als "DNA-Leiter" wahrgenommen werden.

Der Nachweis der Apoptose über FSC/SSC-Analyse kann durch übliche Verfahren erfolgen. Beispielsweise können apoptotische Zellen im Flowzytometer durch charakteristische Veränderungen des Streulicht-Verhaltens (forward scatter/side scatter, FSC/SSC) identifiziert werden.

Der Nachweis der Apoptose über hypodiploide Kerne kann durch übliche Verfahren erfolgen. Beispielsweise werden die mit Arzneistoffen inkubierten Zellen in Nicoletti-Puffer (0,1 Natriumcitrat plus 0,1 % Triton X-100, enthaltend Propidiumjodid 50 µg/ml) lysiert und Propidiumjodid-gefärbte Kerne durch Flowzytometrie (FACScan) analysiert.

Der Nachweis der Apoptose über die Menge von CD95-L mRNA kann durch übliche Verfahren erfolgen. Beispielsweise kann ein Northern Blot durchgeführt werden, bei dem ein Fragment von CD95-L cDNA als spezifische Hybridisierungssonde eingesetzt wird. Eine solche Sonde umfaßt beispielsweise das Fragment 301-899 von CD95-L cDNA.

Quantitativ kann die Menge an CD95-L mRNA durch eine RT-PCR-Reaktion bestimmt werden. Bei der quantitativen PCR für CD95-L wird die Menge von CD95-L cDNA durch Zugabe eines CD95-L spezifischen DNA-Kompetitor-Fragments in die PCR-Reaktion titriert. Diesem Schritt voraus geht die vollständige reverse Transkription einer definierten Menge Gesamt-RNA in cDNA. Das Kompetitor-Fragment unterscheidet sich von der "Wildtyp" cDNA von CD95-L durch zusätzliche Basenpaare in der Mitte der Nukleotidsequenz, besitzt jedoch identische flankierende Sequenzen. Deshalb werden Wildtyp- und Kompetitor-Fragment von der gleichen Primer-Kombination erkannt und können zusammen in einer PCR vervielfältigt werden. Bedingt durch die identischen Primer-Bindungsstellen und die ähnlichen Nukleotidsequenzen unterliegen beide Fragmente der gleichen Amplifikationsrate. Der geringe Größenunterschied erlaubt jedoch die Auftrennung beider DNA-Spezies durch Gelektrophorese nach der PCR. Die Intensitäten der DNA-Banden auf den Agarosegel reflektieren die ursprünglichen Mengen der cDNA, die in der PCR eingesetzt wurden. Wenn die Ausprägung der CD95-L und der Kompetitor-DNA-Bande auf dem Gel identisch ist, kann geschlossen werden, daß von Kompetitor und CD95-L die gleichen Ausgangskonzentrationen eingesetzt wurden. Die Menge der auf diese Weise titrierten CD95-L cDNA spiegelt die Menge der CD95-L mRNA in der Gesamt-RNA wieder, da die reverse Transkription vollständig verläuft.

Das erfindungsgemäße Verfahren zeichnet sich dadurch aus, daß in ihm Konzentrationen von Arzneistoffen auf ihre Aktivität getestet werden können, die auch therapeutisch verabreicht werden. Somit gestatten Ergebnisse, die durch das erfindungsgemäße Verfahren erzielt werden, eine direkte Aussage über die Wirkung von Arzneistoffen beim Patienten.

Ferner bietet das erfindungsgemäße Verfahren neben einer neuen Klassifikation etablierter Arzneistoffe, bevorzugt Zytostatika, eine molekulare Basis zur Entwicklung neuer Arzneistoffe in der Tumor- und AIDS-Therapie, zur Charakterisierung ihrer Nebenwirkungen an normalem Gewebe, z.B. Knochenmark, Immmunsystem, Leber, und zur Diagnostik der Chemotherapieresistenz an Patientenzellen. Darüberhinaus ist mit dem Verfahren ein Ansatz zur Durchbrechung der Zytostatikaresistenz möglich.

### Kurze Beschreibung der Zeichnungen:

- Fig. 1: zeigt die Induktion des CD95-Liganden durch Doxorubicin
- Fig. 2: zeigt die Induktion des CD95-Liganden durch Methotrexat
- Fig. 3: zeigt die Hemmung der Doxorubicin induzierten Apoptose durch CD95 Antikörper

Die vorliegende Erfindung wird durch das folgende Beispiel beschrieben:

### Beispiel: Bestimmung der Induktion des CD95-Liganden durch Zytostatika

Zellen der T-ALL Zellinie CEM wurden mit verschiedenen Konzentrationen Doxorubicin und Methotrexat, jeweils verschieden lange (4 Std., 8 Std., 24 Std.) inkubiert. Aus diesen Zellen wurde unter Verwendung von oligo-dT-Cellulose Gesamt-RNA isoliert. 250 ng dieser Gesamt-RNA wurden mittels reverser Transkriptase gemäß bekannter Verfahren in cDNA umgeschrieben. Zur Bestimmung der Menge von CD95-L mRNA in der Gesamt-RNA wird eine quantitative RT-PCR durchgeführt. Zur Quantifizierung der CD95-L Expressionsrate wird cDNA von je 250 ng revers transkribierter Gesamt-RNA in PCR-Gefäße pipettiert. Jeder Probe mit Ausnahme der Negativkontrolle wird vorstehend beschriebenes, CD95-L-Kompetitor-Fragment in unterschiedlicher Verdünnung von 5 bis 10000 fg zugegeben. Die PCR-Reaktion wird in einem 50µl Ansatz, enthaltend cDNA (=250 ng revers transkribierter Gesamt-RNA) unter Verwendung von 1 x PCR-Puffer (10 mM Tris-HCl, pH 8,8; 50 mM KCI, 0,08 % NP40), 1mM MgCl₂, 200 µM dNTPs, 0,5 µM Primer (Lᵤₚ: 5'-ATCTTCAAACCTCCATGATG-3' = bindet an die Nukleotide 301/325 von menschl. CD95-L; L_{dw}: 5'-CCAGAGAGAGCTCAGATA-CGTTGAC-3' = bindet an die Nukleotide 775/779 von menschl. CD95-L) und 1,12 U/Ansatz Taq-Polymerase durchgeführt.

Die PCR-Reaktionsbedingungen sind: 35 Sek. 94°C, 90 Sek. 56°C und 120 Sek. 72°C, 36 Reaktionszyklen. Jeweils 20 µl der PCR-Reaktions-Mischung werden auf ein Agarosegel aufgetragen, elektrophoretisch aufgetrennt und mit Ethidiumbromid angefärbt. Aus dem Vergleich der Intensitäten der Wildtyp-Bande und der Kompetitor-Bande kann auf die in 250 ng revers transkribierter Gesamt-RNA enthaltene Menge CD95-L mRNA geschlossen werden.

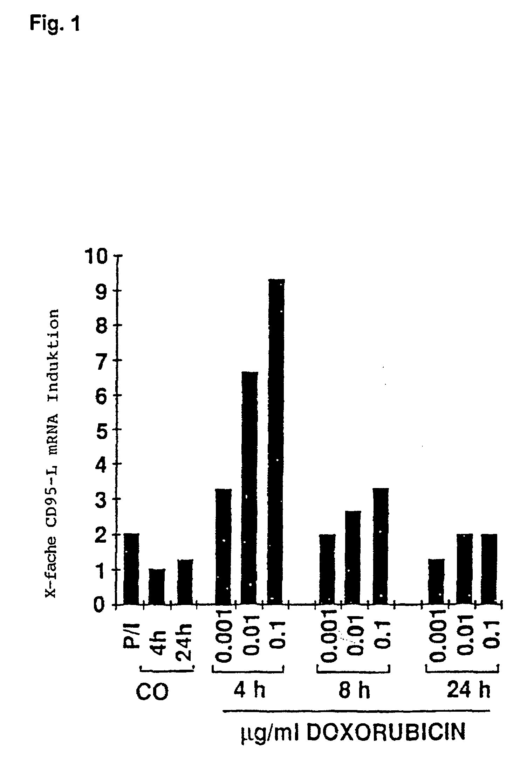

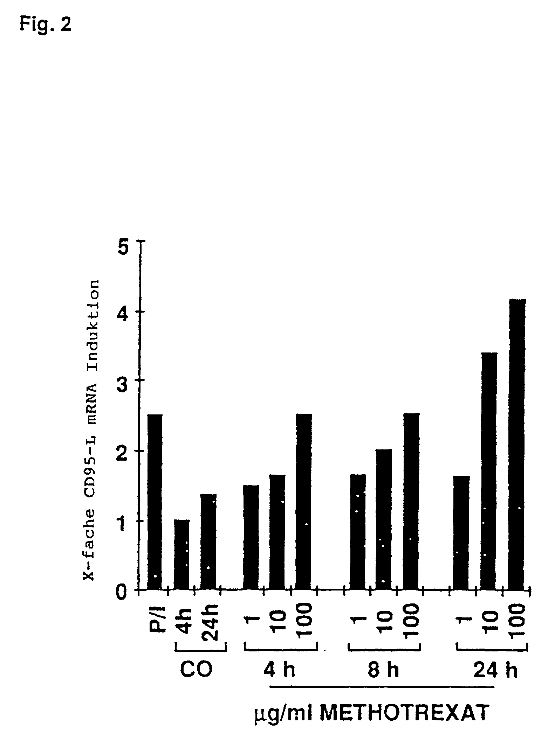

Die mit Doxorubicin und Methotrexat erhaltenen Ergebnisse sind in Fig. 1 und Fig. 2 gezeigt. Daraus geht hervor, daß Doxorubicin und Methotrexat in CDM-Zellen CD95-Ligand induzieren und damit Apoptose anregen.

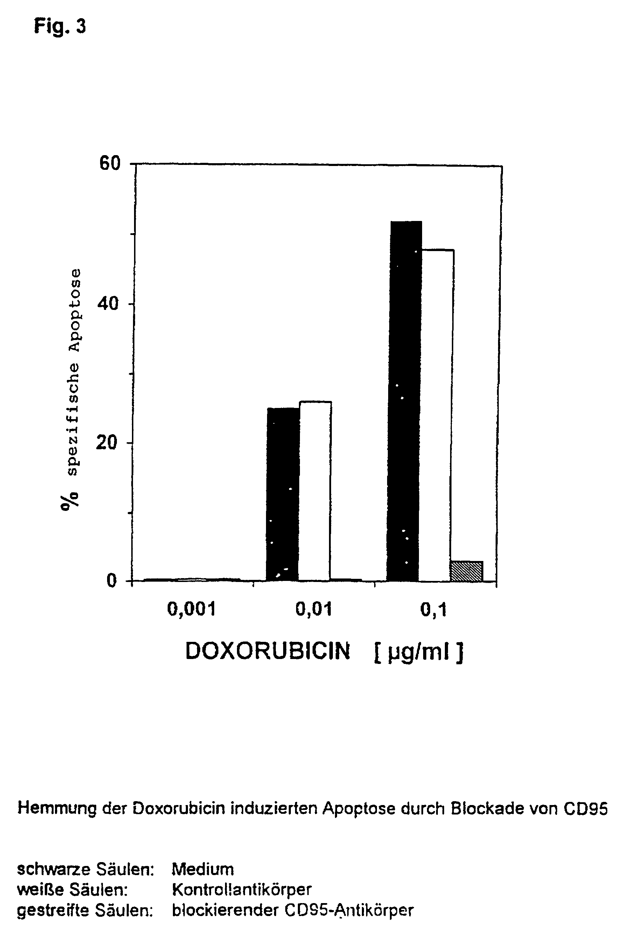

Fig. 3 zeigt, daß die Doxorubicin induzierte Apoptose durch Bindung von CD95-L an den Rezpetor vermittelt wird. Die Blockade von CD95 verhindert die Doxorubicin induzierte Apoptose.

## Patentansprüche

1. Verfahren zur Identifizierung eines zytostatischen Arzneimittels mit CD95-Ligand induzierter zytostatischer Aktivität, umfassend die folgenden Verfahrensschritte:
(a) Bereitstellen von Zellen von denen bekannt ist, daß in ihnen Apoptose als Antwort auf die Expression vom CD95-Ligand ausgelöst wird,
(b) Inkubation dieser Zellen mit einem potentiell zytostatischen Arzneimittel, und
(C) Nachweis des Ausmaßes von Apoptose, die durch Induktion der Expression vom CD95-Ligand über das CD95/CD95-L-System hervorgerufen wird, wodurch das potentiell zytostatische Arzneimittel als ein solches identifiziert wird.

2. Verfahren nach Anspruch 1, **dadurch gekennzeichnet, daß** die Zellen von einer etablierten Zellinie oder einem Patienten stammen.

3. Verfahren nach Anspruch 2, **dadurch gekennzeichnet, daß** die Zellen leukämische Zellen oder Tumorzellen sind.

4. Verfahren nach Anspruch 3, **dadurch gekennzeichnet, daß** die leukämischen Zellen eine T-ALL-Zellinie sind.

5. Verfahren nach Anspruch 4, **dadurch gekennzeichnet, daß** die T-ALL-Zellinie die Zellinie CEM oder Jurkat ist.

6. Verfahren nach einem der Ansprüche 1 bis 5, **dadurch gekennzeichnet, daß** der Nachweis des Ausmaßes der Apoptose erfolgt durch
- morphologische Veränderungen von Zellen,
- DNA-Fragmentationsmuster,
- hypodiploide Kerne,
- FCS/SSC-Analyse
- Bestimmung der Menge von CD95-L mRNA.

7. Verfahren nach Anspruch 6, **dadurch gekennzeichnet, daß** die Bestimmung der mRNA des CD95-Ligand durch Northern-Blot-Analyse oder RT-PCR erfolgt.

## Claims

1. A process for identifying a cytostatic drug having CD95 ligand-induced cytostatic activity, comprising the steps of:
(a) providing cells in which as is known apoptosis is triggered in response to the expression of the CD95 ligand;
(b) incubation of these cells with a potentially cytostatic drug, and
(c) detection of the extent of apoptosis which is caused by the induction of the expression of the CD95 ligand via the CD95/CD95-L system so as to identify the potentially cytostatic drug as such.

2. The process according to claim 1, **characterized in that** the cells originate from an established cell line or a patient.

3. The process according to claim 2, **characterized in that** the cells are leukemic cells or tumor cells.

4. The process according to claim 3, **characterized in that** the leukemic cells are a T-ALL cell line.

5. The process according to claim 4, **characterized in that** the T-ALL cell line is the cell line CEM or Jurkat.

6. The process according to any of claims 1 to 5,
**characterized in that** the extent of apoptosis is detected by
- morphological changes of cells,
- DNA fragmentation patterns,
- hypodiploid nuclei,
- FCS/SSC analysis,
- the detection of the amount of CD95-L mRNA.

7. The process according to claim 6, **characterized in that** the mRNA of the CD95 ligand is detected by Northern blot analysis or RT-PCR.

## Revendications

1. Méthode d'identification d'un médicament cytostatique à activité cytostatique induite par le CD95-ligand, qui comprend les étapes suivantes :
(a) préparation de cellules dont on sait qu'une apoptose est déclenchée en elles comme réponse à l'expression du CD95-ligand,
(b) incubation de ces cellules avec un médicament potentiellement cytostatique, et
(c) détection du degré d'apoptose qui est générée par induction de l'expression du CD95-ligand par l'intermédiaire du système CD95/CD95-L, ce qui permet d'identifier comme tel le médicament potentiellement cytostatique.

2. Méthode suivant la revendication 1, **caractérisée en ce que** les cellules proviennent d'une lignée cellulaire établie ou d'un patient.

3. Méthode suivant la revendication 2, **caractérisée en ce que** les cellules sont des cellules leucémiques ou des cellules tumorales.

4. Méthode suivant la revendication 3, **caractérisée en ce que** les cellules leucémiques consistent en une lignée cellulaire T-ALL.

5. Méthode suivant la revendication 4, **caractérisée en ce que** la lignée cellulaire T-ALL est la lignée cellulaire CEM ou Jurkat.

6. Méthode suivant l'une des revendications 1 à 5, **caractérisée en ce que** la détection du degré d'apoptose est effectuée selon :
- les modifications morphologiques de cellules,
- le modèle de fragmentation de l'ADN,
- les noyaux hypodiploïdes,
- l'analyse FCS/SSC
- la détermination de la quantité d'ARNm du CD95-L.

7. Méthode suivant la revendication 6, **caractérisée en ce que** la détermination de l'ARNm du CD95-ligand est effectuée par analyse Northern Blot ou par PCR avec transcriptase inverse.
